# EUROPÄISCHE PATENTANMELDUNG

(11) **EP 4 687 253 A1**
(43) Veröffentlichungstag der Anmeldung: **04.02.2026**
(21) Anmeldenummer: 24191558.6
(22) Anmeldetag: 29.07.2024
(51) Int. Cl.: H02J 7/00, A61N 1/378

(54) **MEDIZINISCHE FUNKTIONSEINHEIT MIT EINEM ODER MEHREREN NATRIUM-IONEN-AKKUMULATOREN**

(71) Anmelder: Berlin Heart GmbH, 12247 Berlin (DE)
(72) Erfinder: Jahne, Helmut, 12247 Berlin (DE); Bykov, Valentin, 12247 Berlin (DE); Peters, Oliver, 12247 Berlin (DE)
(74) Vertreter: Pfenning, Meinig & Partner mbB

(57) **Zusammenfassung**

Die Erfindung bezieht sich auf eine medizinische Funktionseinheit mit einer elektrischen Energieversorgung, die einen oder mehrere aufladbare Natrium-Ionen-Akkumulatoren (1a) aufweist, wobei die Energieversorgung und die Natrium- Ionen- Akkumulatoren weniger als sechs der folgenden sechs Schutzvorrichtungen oder weniger als 5 oder weniger als 4, weniger als drei, oder weniger als zwei der folgenden sechs Schutzvorrichtungen (7) aufweisen: Ladestromschutz, Laststromschutz, Unterspannungsschutz, Überladungsschutz, Hochtemperaturschutz, temperaturabhängige Lastrombegrenzung, sowie auf ein Verfahren zur Sterilisierung einer Funktionseinheit mit Natrium- Ionen- Akkumulatoren.

## Beschreibung

Die Erfindung liegt auf dem Gebiet der Elektrotechnik und der Medizintechnik, und ist mit besonderem Vorteil bei transportablen Systemen der Medizintechnik, beispielsweise bei transportablen Herzunterstützungssystemen, einsetzbar.

In elektronischen Geräten, insbesondere in tragbaren Geräten der Medizintechnik und ganz besonders in tragbaren Herz-Unterstützungssystemen, werden bisher stets Batterien, insbesondere wiederaufladbare Batterien/Ackus/Akkumulatoren, mit möglichst hoher Energiedichte eingesetzt. Eine hohe Energiedichte der eingesetzten Batterien bedeutet, dass die zum Betrieb der elektronischen Geräte erforderliche Energie aus einem kleinen Volumen und/oder aus einer Stromversorgung mit geringem Gewicht zur Verfügung gestellt wird. Dies führt zu kleinen Geräten und langer Akkulaufzeit. Insbesondere bei elektronischen medizinischen Geräten werden bisher weit überwiegend relativ teure Lithium-lonen-Akkus eingesetzt.

Dass bei hochwertigen elektronischen Geräten in der Medizin, Die Verwendung von Batterien mit sehr hoher Energiedichte für hochwertige medizinische Geräte geht unter anderem aus den US-Patentdokumenten US8394009B2 und US8585571B2 hervor. Diese Dokumente beschreiben in verschiedenen Konfigurationen den Einsatz von Batterien mit hoher Energiedichte in Herzunterstützungssystemen. Der Fokus liegt dabei jeweils auf der Verwendung von Batterien mit hoher Energiedichte.

Nachteilig beim Einsatz von Lithium-Ionen Akkus/Akkumulatoren in tragbaren elektronischen Geräten ist beispielsweise, dass Lithium-Ionen-Akkus grundsätzlich die Eigenschaft besitzen, dass sie unter bestimmten Bedingungen thermisch durchgehen können, das heißt, dass in warmen Akku-Zellen eine Kettenreaktion in Gang gesetzt werden kann, indem eine chemische Reaktion einsetzt, die diese Zellen ohne äußere Einflüsse weiter aufheizt. Weiter besteht das Risiko, dass Lithium-Ionen-Akkus brennen und explodieren können. Um diese Risiken bei der Verwendung gering zu halten, müssen zum sicheren Betrieb von Lithium-Ionen-Akkus einige Sicherheitsmechanismen/Schutzfunktionen oder Schutzvorrichtungen vorgesehen werden. Die meisten Schutzfunktionen übernimmt hierbei üblicherweise ein Batterie-Management-System (BMS). Das BMS schützt den Lithium-Ionen-Akku vor zu hohen Akku-Spannungen, wenn z.B. die Ladeschaltung fehlerbehaftet ist, es schützt vor zu tiefen Entladungen und vor zu großen Ladeströmen, vor zu großen Entladeströmen und vor Kurzschlüssen, wenn z.B. die Anwendungsschaltung keinen solchen Schutz vorsieht und es unterbricht die Ladung der Akkuzellen bei zu hohen Zell-Temperaturen. Darüber hinaus kann ein BMS noch weitere Funktionen übernehmen, wie z.B. die Ermittlung des genauen Ladezustandes oder auch den Ladungsausgleich zwischen verschiedenen Akku-Zellen, wenn mehrere Zellen in Reihe geschaltet sind.

Die Gefahr, dass Lithium-Ionen-Zellen thermisch durchgehen können, also eine Kettenreaktion in Gang setzen können, indem in warmen Akku-Zellen eine chemische Reaktion einsetzt, die diese Zelle ohne äußere Einflüsse weiter aufheizt, führt dazu, dass die Einsatztemperaturen von Lithium-Ionen-Akkus begrenzt sind. Es ist sowohl die zulässige Temperatur bei der Nutzung von mit Lithium-Ionen-Akkus betriebenen Geräten als auch die zulässige Temperatur bei der Aufladung der Akkus in diesen Geräten begrenzt. Typische Grenzen sind in der DIN-EN 62133-2 genannt. In der Regel ist das Entladen von Lithium-lonen-Zellen auf Temperaturen unter 45 °C und das Aufladen auf Temperaturen unter 40 °C begrenzt. Das schränkt beispielsweise bereits den Einsatz von tragbaren medizinischen Geräten insbesondere von Herzunterstützungs-Geräten mit Lithium-Ionen-Akkus im Freien bei direkter Sonnen- Einstrahlung und/oder mit Körperkontakt deutlich ein.

Lithium-Ionen-Akkumulatoren weisen zudem eine deutliche Kapazitätsreduzierung bei niedrigeren Temperaturen auf. Der Nutzer eines elektronischen Geräts mit einem Lithium-lonen-Akku muss also bei der Nutzung in kalten Umgebungen, bedingt durch Kapazitätseinbußen, eine deutliche Laufzeitreduzierung hinnehmen.

Da als eine der Schutzfunktonen bei Lithium-Ionen-Akkus üblicherweise eine Strombegrenzung vorgesehen ist, besteht auch die Gefahr, dass in Geräten, die sehr hohe Spitzenströme für die ordnungsgemäße Funktion erfordern, z.B. bei Herzunterstützungssystemen, diese Spitzenströme nicht zur Verfügung gestellt werden können, wenn die Schutzfunktion diese zum Schutz der Lithium-Ionen-Akkus begrenzen muss. Die Begrenzung von Spitzenströmen kann dazu führen, dass besonders hochstromfeste Lithium-Ionen-Akkus verwendet werden müssen, die auf Kosten der Kapazität besonders hohe Spitzenströme zulassen. Dadurch steht für das betreffende elektronische Gerät dann nur eine geringere Batterie-Kapazität zur Verfügung.

Darüber hinaus erfordern Lithium-Ionen-Akkus immer eine Mindest-Akku-Spannung, da unterhalb dieser minimalen Spannung die Zellen irreversibel geschädigt werden und die Gefahr einer Selbstentzündung besteht. Deshalb dürfen solche Akkus niemals vollständig entladen werden und die Einhaltung der Mindest-Akku-Spannung muss auch bei Nicht-Benutzung, also beispielsweise bei Lagerung, zyklisch überwacht werden. Diese Mindestspannung verhindert eine übliche Sterilisation von Lithium-Ionen-Akkus und von Geräten, welche solche Akkuzellen enthalten, da die Sterilisation oft unter Einsatz von reaktiven Gasen erfolgt, die unter dem Einfluss von elektrischen Spannungen brennbar oder explosiv sein können. Solche Akkus müssen daher wechselbar sein und können erst außerhalb eines Sterilbereiches eingesetzt werden. Weiterhin können steril verpackte medizinische Geräte, die übliche Lithium-Ionen-Akkus enthalten, nicht über längere Zeit gelagert werden , da eine zyklische Überprüfung des Ladungszustandes und gegebenenfalls ein Nachladen beispielsweise in einer Sterilverpackung nicht möglich ist.

Ein weiteres Problem von Lithium-Ionen-Akkus besteht darin, dass bei den Ausführungen mit hoher Energiedichte neben Lithium auch Kobalt und Nickel zur Herstellung benötigt werden, die als Rohstoffe auf der Erde nicht in gro-ßen Mengen verfügbar oder kaum ohne Umweltschäden abbaubar sind.

Aus dem Dokument WO2016163473A1 ist weiterhin bekannt, dass in medizinischen Geräten auch Akku-Zellen eingesetzt werden können, die nicht auf einer Lithium-Ionen-Technologie basieren und andere Materialien verwenden. Allerdings wird dort auf die besonderen Eigenschaften einzelner Akku-Technologien nicht eingegangen.

Der vorliegenden Erfindung liegt vor dem Hintergrund des Standes der Technik die Aufgabe zugrunde, für medizinische Funktionseinheiten, beispielsweise als Teil von Herzunterstützungssystemen, eine Energieversorgung zu schaffen, die die Nachteile von Lithium-Ionen-Akkus wenigstens teilweise vermeidet.

Die Aufgabe wird mit den Merkmalen der unabhängigen Patentansprüche gelöst. In den abhängigen Patentansprüchen sind mögliche Implementierungen vorgestellt.

Die Erfindung bezieht sich somit auf eine medizinische Funktionseinheit mit einer elektrischen Energieversorgung, die einen oder mehrere aufladbare Natrium-lonen-Akkumulatoren aufweist, wobei die Energieversorgung und die Natrium- lonen- Akkumulatoren weniger als sechs der folgenden sechs Schutzvorrichtungen oder weniger als 5 oder weniger als 4, weniger als drei, oder weniger als zwei der folgenden sechs Schutzvorrichtungen aufweisen:
Ladestromschutz (Definition siehe am Ende dieses Textes),
Laststromschutz,
Unterspannungsschutz,
Überladungsschutz,
Hochtemperaturschutz,
temperaturabhängige Lastrombegrenzung.

Dabei können die genannten Schutzvorrichtungen beispielsweise wie folgt definiert werden:
Der Ladestromschutz ist eine Vorrichtung, die den Ladestrom begrenzt und/oder bei einem zu hohen Ladestrom, also bei Überschreiten einer kritischen Schwelle, diesen abschaltet.

Der Laststromschutz ist eine Vorrichtung, die den Laststrom überwacht und diesen begrenzt oder bei Überschreiten einer kritischen Schwelle abschaltet.

Der Unterspannungsschutz ist eine Vorrichtung, die, falls die Akkuspannung eine Mindestschwelle unterschreitet, Gegenmaßnahmen einleitet (z.B. Abschalten einer Last, Wiederaufladen durch eine zur Verfügung stehende Energiequelle oder dauerhaftes Abtrennen der Zellen, um eine Wiederaufladung zu verhindern) und/oder ein Warnsignal abgibt oder abspeichert.

Der Überladungsschutz ist eine Vorrichtung, die die Zellenspannung überwacht und einen Ladevorgang bei Überschreiten einer Spannungsschwelle abbricht/den Ladestrom unterbricht.

Der Hochtemperaturschutz ist eine Vorrichtung, die die Zellentemperatur überwacht und beim Entladen bei Überschreiten einer ersten Temperaturschwelle den Entladungsstrom unterbricht und/oder die beim Laden bei Überschreiten einer zweiten Temperaturschwelle den Ladestrom unterbricht oder begrenzt.

Eine temperaturabhängige Lastrombegrenzung ist eine Vorrichtung, die bei einer Entladung die Temperatur der Zellen überwacht und in Abhängigkeit von der Temperatur der Zellen den Laststrom begrenzt.

Natrium-Ionen-Akkus benötigen die genannten Schutzvorrichtungen nicht, sodass mehrere oder alle genannten Schutzvorrichtungen bei Natrium-Ionen-Akkus und bei Funktionseinheiten oder Geräten, die mit Natrium-Ionen-Akkus ausgestattet werden, nicht vorgesehen werden müssen. Hierdurch ergibt sich eine beträchtliche Einsparung an Bauraum und Gewicht, sodass die Brutto-Speicherdichte für Energie d. h. die Volumen- oder Massebezogene Energie, die in einem Einheitsvolumen oder pro Masseneinheit bei Berücksichtigung notwendiger Schutzvorrichtungen gespeichert werden kann, für Natrium-lonen-Akkus konkurrenzfähig mit der Brutto-Speicherdichte von Lithium-Ionen-Akkus oder sogar besser als diese ist.

Die Einsparung von Schutzvorrichtungen spart insgesamt nicht nur Bauraum und Masse sondern auch Kosten. Zudem ist durch eine Vereinfachung der Anwendung von Akkumulatoren bei Verwendung von Natrium-Ionen-Akkumulatoren eine höhere Ausfallsicherheit gegeben, da die Zahl und der Umfang von Schutzvorrichtungen, die auch fehlerbehaftet sein können, reduziert oder auf 0 minimiert werden kann.

Es liegt ein wesentlicher Vorteil der Erfindung darin, dass in einem elektronischen medizinischen Gerät an Stelle von Lithium-Ionen-Akkus, Natrium-Ionen Akkus zum Einsatz kommen. Natrium-lonen-Akkus besitzen pro Zelle betrachtet zwar nur etwa die halbe Energiedichte gegenüber Lithium-lonen-Akkus, so dass allgemein bisher erwartet wurde, dass tragbare elektronische Geräte, die Natrium-lonen-Akkus verwenden, größer sein müssten als funktionsgleiche Geräte mit Lithium-lonen-Akkus. Tatsächlich besitzen Natrium-Ionen-Akkus jedoch Eigenschaften, die sich von den Lithium-Ionen-Akkus grundlegend unterscheiden, so dass bei einem Verzicht auf Lithium-Ionen-Akkus auch erhebliche Einsparungen an Volumen und Gewicht in den sie enthaltenden Geräten durch den Verzicht auf Schutzvorrichtungen realisiert werden können.

Das Wesen der Erfindung besteht in der Berücksichtigung der Brutto-Energiedichte eines Akkus anstelle der bisher üblichen Energiedichte der eingesetzten Akku-Zellen, der Netto-Energiedichte.

Für ein tragbares elektronisches Gerät, welches mit Akkumulatoren betrieben wird, ist nicht die Energiedichte der Akku-Zelle maßgebend, sondern die Brutto-Energiedichte, welche die Akkuzelle mit all ihren erforderlichen Schutzvorrichtungen/Schutzschaltungen und Zusatzeinrichtungen enthält. Dieser Umstand ist bisher nicht beachtet worden.

Ein weiterer Vorteil bei der Nutzung des Natrium Ionen Akkus, ist die bessere Kapazitätsnutzung über den zulässigen Temperaturbereich (-30 °C bis +6O °C). Natrium-Ionen Akkumulatoren weisen eine deutlich geringere Kapazitätsreduzierung bei niedrigeren Temperaturen auf als Lithium-Ionen Akkumulatoren. Dies stellt eine erhebliche Verbesserung der Brauchbarkeit dar.

Oft sind gemäß dem Stand der Technik im Zusammenhang mit Akkumulatoren noch weitere Funktionen vorgesehen. Oft ist ein Batteriemanagementsystem vorgesehen, das auch die Übermittlung von Daten von und zu den Akkumulatoren übernimmt. So kann vielfach von einem Mikroprozessor aus mit dem Batteriemanagement-System kommuniziert werden. Das BMS kann z.B. den aktuellen Ladungszustand der Akku-Zellen feststellen und diesen über einen Kommunikationsbus einem Mikroprozessor mitteilen. Dies ist bei Akku-Zellen mit flacher Lade- und Endladekennlinie, wie z.B. bei Lithium-Ionen-Akkus, insbesondere Lithium-Eisen-Phosphat-Akkus, sehr hilfreich, da andere Methoden hier nur sehr ungenaue Ergebnisse liefern.

Natrium-Ionen-Akkumulatoren hingegen weisen eine steilere Lade- und Endladekennlinie auf, so dass allein über die Messung der Zellspannung eine relativ gute Aussage über den Ladezustand der Akku-Zellen möglich ist.

Somit kann bei einem Natrium-Ionen-Akkumulator auf ein Batteriemanagementsystem oft vollständig verzichtet werden. Lediglich bei Akkupacks/Akkumulatorkombinationen mit mehreren in Reihe geschalteten Akkuzellen kann sowohl bei Lithium-Ionen Akkus als auch bei Natrium-Ionen-Akkus ein Ladungsausgleich zwischen den einzelnen Zellen vorgesehen sein.

Mit dem Wegfall eines üblichen Batteriemanagementsystems kann bei Verwendung von Natrium-Ionen-Akkus auch die Strombegrenzungsfunktion entfallen. Daher können Geräten, die sehr hohe Spitzenströme für die ordnungsgemäße Funktion erfordern, z.B. bei Herzunterstützungssysteme, uneingeschränkt betrieben werden. Bei Natrium-Ionen-Akkus können zulässige Grenzen des Lade- oder Entladestroms zumindest kurzfristig auch ohne ein Brandrisiko überschritten werden. Es muss für die jeweilige Anwendung kein Kompromiss zwischen hochstromfesten Batterie-Zellen/Akkumulatoren und solchen Zellen/Akkumulatoren mit großer Kapazität gefunden werden. Beide Eigenschaften sind in demselben Akkumulator vereint.

Da bei einem Natrium-Ionen-Akkumulator auf ein Batteriemanagementsystem verzichtet werden kann, entfallen auch die Kommunikation mit einem Prozessor sowie die entsprechenden Steckkontakte an einem Akku-Pack. Die Baugröße von Akkumulatoren kann bei Verwendung von Natrium- lonen-Akkumulatoren im Vergleich mit anderen Akkumulatorbauarten bei gleicher Kapazität verringert werden oder bei gleicher Baugröße steht eine vergrößerte Kapazität zur Verfügung.

Bei einem Natrium-Ionen-Akku sind die Brutto-Energiedichte und die Netto-Energiedichte der Zellen gleich groß, weil keine für diese Akku-Zellen notwendigen sicherheitsrelevanten Zusatzeinrichtungen erforderlich sind. Daher können tragbare elektronische Geräte mit Natrium-Ionen-Akkumulatoren bei gleicher Akku-Laufzeit, trotz der größeren Zellen-Geometrie kleiner oder gleich groß sein,
Es kann in einer vorteilhaften Ausführungsform beispielsweise vorgesehen sein, dass bei einer Funktionseinheit der beschriebenen Art die Kathode oder die Kathoden und die Anode oder die Anoden der Natrium- lonen- Akkumulatoren unmittelbar mit einer Ladespannungsquelle verbunden oder verbindbar sind.

Eine unmittelbare Verbindung der Anschlüsse von Natrium-Ionen-Akkumulatoren mit einer Ladungsspannungsquelle kann bedeuten, dass keine Schutzvorrichtung elektrisch in Reihe geschaltet mit den Akkumulatoren zwischen diesen und der Ladevorrichtung vorgesehen ist. Eine unmittelbare Verbindung kann in diesem Zusammenhang auch bedeuten, dass die Anschlüsse der Natrium-lonen-Akkus ohne Einbeziehung von Schutzvorrichtungen oder ohne Zwischenschaltung oder Einbeziehung eines Batteriemanagementsystems mit einer Ladespannungsquelle verbunden ist. Dies gilt unabhängig davon, welche Schutzvorrichtungen in der Ladevorrichtung selbst vorgesehen sind. Das Einfügen von Natrium-Ionen- Akkus in eine Funktionseinheit oder ein elektrisches Gerät ist somit mit einem sehr geringen Aufwand verbunden. Die Ladespannungsquelle kann beispielsweise mit der Anwendungselektronik verbunden sein, mit der ihrerseits die natrium-lonen- Akkus unmittelbar verbunden sein können.

Es kann zudem vorgesehen sein, dass die Ladespannungsquelle durch einen induktiven Ladekreis gebildet ist.

Bei vielen Geräten, die mit Akkumulatoren betrieben werden, ist eine Aufladung ohne Herstellung einer galvanischen Verbindung über ein Magnetfeld durch induktives Laden möglich. Dies kann insbesondere für implantierbare Funktionseinheiten der Medizintechnik attraktiv sein, da beim induktiven Laden keine transkutane Verbindung zu den aufzuladenden Zellen notwendig ist und somit die Risiken von Infektionen durch transkutane Verbindungen minimiert werden können.

Es kann auch vorgesehen sein, dass bei einer Funktionseinheit der beschriebenen Art die Kathode oder die Kathoden und die Anode oder die Anoden der Natrium- lonen- Akkumulatoren unmittelbar mit einem elektrischen Aggregat verbunden oder verbindbar sind, das mit der Energie aus dem oder den Natrium- lonen- Akkumulatoren betreibbar ist.

Eine unmittelbare Verbindung der Anschlüsse von Natrium-Ionen-Akkumulatoren mit einem elektrischen Aggregat, das mit der Energie aus dem oder den Natrium- lonen- Akkumulatoren betreibbar ist kann bedeuten, dass keine Schutzvorrichtung elektrisch in Reihe geschaltet mit den Akkumulatoren zwischen diesen und dem elektrischen Aggregat vorgesehen ist. Dies gilt unabhängig davon, welche Schutzvorrichtungen in dem elektrischen Aggregat selbst vorgesehen sind. Eine unmittelbare Verbindung kann in diesem Zusammenhang auch bedeuten, dass die Anschlüsse der Natrium-Ionen-Akkus ohne Einbeziehung von Schutzvorrichtungen oder ohne Zwischenschaltung oder Einbeziehung eines Batteriemanagementsystems mit einem elektrischen Aggregat verbunden ist. Das Einfügen von Natrium-Ionen- Akkus in eine Funktionseinheit oder ein elektrisches Gerät ist somit mit einem sehr geringen Aufwand verbunden.

Eine weitere, besondere Ausführungsform kann beispielsweise vorsehen, dass bei einer Funktionseinheit der beschriebenen Art die Energieversorgung und die Natrium- lonen- Akkumulatoren keine der folgenden sechs Schutzvorrichtungen aufweisen:
Ladestromschutz,
Laststromschutz,
Unterspannungsschutz,
Überladungsschutz,
Hochtemperaturschutz,
temperaturabhängige Lastrombegrenzung
und dass die Kathode oder die Kathoden und die Anode oder die Anoden der Natrium- lonen- Akkumulatoren unmittelbar mit einer Ladespannungsquelle verbunden oder verbindbar sind.

Es sei zudem angemerkt, dass auch in diesem Fall die Ladespannungsquelle in einigen Fällen durch einen induktiven Ladekreis gebildet sein kann.

Es kann bei einer Funktionseinheit der beschriebenen Art auch vorgesehen sein, dass die Energieversorgung und die Natrium- lonen- Akkumulatoren keine der folgenden sechs Schutzvorrichtungen aufweisen:
Ladestromschutz,
Laststromschutz,
Unterspannungsschutz,
Überladungsschutz,
Hochtemperaturschutz,
temperaturabhängige Lastrombegrenzung
und dass die Kathode oder die Kathoden und die Anode oder die Anoden der Natrium- lonen- Akkumulatoren unmittelbar mit einem elektrischen Aggregat verbunden oder verbindbar sind, das mit der Energie aus dem oder den Natrium- lonen- Akkumulatoren betreibbar ist.

Die beiden letztgenannten Ausführungsformen machen deutlich, dass eine Funktionseinheit mit Natrium-Ionen-Akkus nicht nur mit weniger als sechs der genannten Schutzvorrichtungen, sondern auch gänzlich ohne eine der genannten Schutzvorrichtungen auskommen kann und die natrium-lonen-Akkus in einfacher Weise in die Funktionseinheit integriert und an diese angeschlossen werden können. Dies ist, wie bereits weiter oben angedeutet, auch möglich, wenn keine der genannten Schutzvorrichtungen anderweitig in der Funktionseinheit vorgesehen ist.

Eine weitere Ausführungsform kann bei einer Funktionseinheit der beschriebenen Art auch vorsehen, dass die Energieversorgung und die Natrium- lonen-Akkumulatoren keine Schutzvorrichtung aufweisen, die während einer Lagerung Messgrößen der Natrium- lonen- Akkumulatoren erfasst und/oder ein Warnsignal bei Unterschreiten einer Spannungsschwelle abgibt oder abspeichert.

Wie bereits ausgeführt wurde, ist die Lagerung von bisher in großem Umfang verwendeten Lithium-Ionen-Akkus wegen eines möglichen Unterschreitens einer Unterspannungsschwelle riskant. Unterhalb dieser Schwelle können chemische Reaktionen in Gang gesetzt werden, die ohne äußere Einflüsse den Akku durch Brand oder Explosion zerstören können und dabei auch andere Aggregate und das Umfeld erheblich gefährden. Aus diesem Grund müssen beispielsweise Lithium-lonen-Akkus bezüglich der Zellenspannung bei Lagerung überwacht werden. Hierzu sind Überwachungseinrichtungen bekannt, die ein Alarmsignal abgeben, sobald die Spannung einer Zelle eine Unterspannungsschwelle unterschreitet. Voraussetzung für ein verlässliches Lagern ist dabei, dass auf ein Warnsignal auch Gegenmaßnahmen getroffen werden können.

Bei der Lagerung von Natrium- lonen- Akkus tritt das genannte Unterspannungsproblem und ein entsprechendes Risiko nicht auf, so dass Natrium-lonen- Akkus in einfacher Weise unaufwändig und ohne Schutzvorrichtungen und Überwachungseinrichtungen gelagert werden können. Dieser Umstand macht eine Lagerung und Verteilung von Natrium-lonen-Akkus unabhängig von Zeitplänen möglich, auch wenn die Akkus beispielsweise bereits in verpackte Funktionseinheiten eingebaut sind und nicht nachgeladen werden können.

Es kann in einer weiteren Ausführungsform vorgesehen sein, dass die medizinische Funktionseinheit Teil eines ventrikulären Herzunterstützungsgerätes (VAD) ist.

Ventrikuläre Herzunterstützungsgeräte und -Systeme erfordern eine extrem zuverlässige und ausfallsichere Energieversorgung, da bei einem Ausfall der Versorgung die Gesundheit eines Patienten unmittelbar gefährdet ist. Gleichzeitig sollen solche Herzunterstützungsgeräte jedoch die Mobilität der Patienten möglichst wenig einschränken und sollen deshalb so klein und leicht wie möglich gebaut werden können. Eine hohe Brutto- Energiedichte der zur mobilen Energieversorgung benötigten Akkumulatoren einschließlich der notwendigen Schutzvorrichtungen, verbunden mit einer hohen Ausfallsicherheit aufgrund einer intrinsischen Sicherheit der Akkumulatorzellen, ist somit gerade für Herzunterstützungsgeräte extrem wertvoll.

Zudem kann vorgesehen sein, dass das ventrikuläre Herzunterstützungsgerät einen implantierbaren Teil und einen extrakorporalen Teil aufweist und dass die Natrium- lonen- Akkumulatoren in dem extrakorporalen Teil angeordnet sind oder dass die Natrium- lonen- Akkumulatoren in dem implantierbaren Teil angeordnet sind.

Im Einzelfall kann es vorteilhaft sein, wenn Natrium-Ionen-Akkumulatoren in dem extrakorporalen Teil eines Herzunterstützungsgerätes angeordnet sind. In diesem Teil können sie einfach nachgeladen werden und sind einfach zugänglich.

In anderen Fällen kann auch die Verwendung von Natrium-Ionen-Akkumulatoren in dem implantierbaren oder implantierten Teil von Herzunterstützungsgeräten sinnvoll sein, wenn beispielsweise mit der in den Akkumulatoren gespeicherten Energie unmittelbar eine implantierte Blutpumpe angetrieben werden soll.

Es kann auch vorgesehen sein, dass die Natrium- lonen- Akkumulatoren mittels eines oder mehrerer Steckverbinder mit dem elektrischen Teil der medizinischen Funktionseinheit verbindbar sind oder
dass die Natrium- lonen- Akkumulatoren in einen Batterieschacht der medizinischen Funktionseinheit einsetzbar und in diesem mittels federnder Drucckontakte kontaktierbar sind.

Da der oder die Natrium-Ionen-Akkus ohne weitere Schutzvorrichtungen einsetzbar sind, können diese einfach in eine Funktionseinheit oder ein Gerät eingesetzt werden. Sie können, da sie ohne Risiko vollständig entladen werden können, auch gut und ohne größeren Aufwand sterilisiert werden und im sterilen Zustand in eine Funktionseinheit eingesetzt werden, dabei ist eine einfache Kontaktierung hilfreich, da somit beim Einsetzen der Akkus die Gefahr einer Kontamination minimiert ist. Es ist jedoch in vielen Fällen auch eine Sterilisation der Akkus gemeinsam mit einer Funktionseinheit und/oder einem Gerät möglich.

Eine weitere Realisierungsmöglichkeit kann vorsehen, dass die Natrium- lonen- Akkumulatoren in einem Gehäuse der medizinischen Funktionseinheit fest verbaut sind.

Die Eigenschaft der Akkumulatoren, fest verbaut zu sein, kann beispielsweise bedeuten, dass sie in einem Gehäuse derart verbaut sind, dass sie nicht ohne Spezialwerkzeug/nicht ohne ein über einen Schraubenzieher hinausgehendes Werkzeug/ nur durch Fachpersonal/nur unter Zerstörung von stoffschlüssigen Verbindungen oder nicht reversibel aus dem Gehäuse ausbaubar oder dem Gehäuse entnehmbar sind. Fest verbaute Akku-Zellen haben den Vorteil, dass keine Steckverbinder erforderlich sind und eventuelle Öffnungen zum Wechseln der Akkus entfallen können. Hierdurch wird die Zuverlässigkeit erhöht, da Steckkontakte oder der Innenraum des Medizingeräts durch Öffnungen kontaminiert werden können und später korrodieren können. Ein fester Einbau der Stromversorgung ermöglicht ein hermetisch geschlossenes Gehäuse ohne Öffnungen.

Eine solche Integration der Akkus in eine Funktionseinheit ist unter anderem deshalb möglich, weil eine längere Lagerung der Funktionseinheit gemeinsam mit den Akkus ohne eine Überwachung möglich ist. Zudem kann bei medizinischen Anwendungen eine Sterilisation der Akkus gemeinsam mit der Funktionseinheit vorgenommen werden, da die Akkus gefahrlos vorher vollständig entladen werden können. Ein vollständiges Entladen der Akkus kann in diesem Zusammenhang bedeuten, dass diese über längere Zeit, das heißt über Tage oder Wochen kurzgeschlossen gelagert werden und keine messbare Restspannung mehr vorhanden ist.

Die Erfindung bezieht sich zudem auf eine medizinische Funktionseinheit der oben beschriebenen Art, beispielsweise ein Steuergerät für eine Herzunterstützungsvorrichtung, mit einer die Funktionseinheit gemeinsam mit einem oder mehreren Natrium- lonen-Akkus einschließenden Sterilverpackung.

Diese Kombination ist mit Lithium,-lonen- Akkus nicht sinnvoll, da diese ständig überwacht und bei Erreichen einer Unterspannungsschwelle nachgeladen werden müssen. Bei Verwendung von Natrium- lonen- Akkuzellen bedarf es weder einer Überwachung, noch des Nachladens, so dass die Funktionseinheit, beispielsweise in Form eines Steuergerätes eines Herzunterstützungssystems, problemlos in der Sterilverpackung über längere Zeit gelagert und transportiert werden kann. Die Sterilverpackung kann beispielsweise aus einem hermetisch verschweißten flexiblen Kunststoffbeutel bestehen.

Die Erfindung kann sich auch auf ein ventrikuläres Herzunterstützungssystem mit einer medizinischen Funktionseinheit der oben beschriebenen Art beziehen.

Wie oben bereits beschrieben, kann dann beispielsweise in einem implantierbaren oder in einem extrakorporalen Teil des Herzunterstützungssystems eine Funktionseinheit, beispielsweise eine Steuereinheit oder eine Energieversorgung für einen Pumpenantrieb mit Natrium-lonen-Akkus ausgerüstet sein.

Außer auf eine Funktionseinheit der oben beschriebenen Art und ein ventrikuläres Herzunterstützungssystem bezieht sich die Erfindung auch auf ein Verfahren zur Sterilisation eines oder mehrerer Natrium- lonen-Akkumulatoren dadurch gekennzeichnet, dass der oder die Natrium- lonen- Akkumulatoren zunächst vollständig entladen und nach dem Entladen sterilisiert werden.

Bei bisher beispielsweise für transportable Geräte üblichen Akkumulatoren ist die Sterilisation sehr aufwändig, da sie bei Vorhandensein einer Restspannung der Akkumulatoren durchgeführt werden muss. Manche Sterilisationsverfahren erfordern auch eine Aufheizung der Akkus. Aus diesem Grunde scheidet eine Anzahl von Sterilisationsverfahren für viele Bauarten von Akkumulatoren aus. Mit Natrium-Ionen-Akkus sind der Art der Sterilisation keine Grenzen gesetzt, da sie robust sind, hohe Temperaturen vertragen und vollständig entladen werden können.

Bei einem Verfahren der genannten Art kann auch vorgesehen sein, dass zur Sterilisation einer Funktionseinheit mit einem oder mehreren Natrium- lonen-Akkumulatoren die Natrium- lonen- Akkumulatoren, mit denen die Funktionseinheit betrieben wird, vor oder nach dem Einsetzen in die Funktionseinheit vollständig entladen werden und dass nach dem Entladen der Natrium- lonen-Akkumulatoren die Funktionseinheit gemeinsam mit den Natrium- lonen- Ackumulatoren sterilisiert wird.

In einem Sterilisationsverfahren kann somit mit geringem Aufwand die Funktionseinheit gemeinsam mit den Akkus sterilisiert werden, wobei vorher die Ackus in dem oder außerhalb der Funktionseinheit entladen werden können.

Zudem kann bei einem Verfahren der oben beschriebenen Art auch vorgesehen sein, dass zur Sterilisation einer, insbesondere implantierbaren, medizinischen Funktionseinheit die Natrium- lonen- Akkumulatoren, mit denen die medizinische Funktionseinheit betrieben wird, vor oder nach dem Einsetzen oder dem festen Einbau in die medizinische Funktionseinheit vollständig entladen werden und dass nach dem Entladen der Natrium- Ionen- Akkumulatoren die medizinische Funktionseinheit gemeinsam mit den Natrium- lonen-Akkumulatoren sterilisiert wird.

Der oben beschriebene Sterilisationsprozess entfaltet viele seiner Vorteile insbesondere bei der Sterilisation von medizinischen Funktionseinheiten, die mit Patienten in Berührung kommen oder sogar ganz oder teilweise implantiert werden.

Ein elektronisches Gerät kann mit eingebauten Natrium-Ionen-Akkus sterilisiert werden und es kann in einer Sterilverpackung ohne zyklische Überwachung Jahrelang gelagert werden. Beides ist nur dadurch möglich, dass eine vollständige Entladung von Natrium-Ionen-Akkuzellen möglich ist, ohne dass die Akkuzellen dadurch einen Schaden erleiden. Das ermöglicht es beispielsweise, Natrium-lonen-Akkus bereits bei der Elektronik-Fertigung zu bestücken.

Bei Herzunterstützungssystemen war es bisher während einer Herzoperation üblich, elektronische Geräte im Sterilbereich über Kabel aus anderen Energiequellen außerhalb des Sterilbereichs mit elektrischem Strom zu versorgen. Erst nach einer erfolgreichen Operation konnte dann ein nicht steriler Akku eingesetzt werden. Hierzu war bei dem betreffenden elektronischen Gerät im Gehäuse eine Öffnung zum Einbringen eines Akkus vorzusehen. Diese Gehäuseöffnung musste flüssigkeitsdicht verschließbar gestaltet sein. Der zusätzlich erforderliche Bauraum für eine flüssigkeitsdicht verschließbare Öffnung kann durch die Verwendung von Natrium-lonen-Akkus eingespart werden.

Im Folgenden wird die Erfindung anhand von Ausführungsbeispielen in Figuren einer Zeichnung gezeigt und nachfolgend beschrieben.

Dabei zeigt
- Figur 1:: schematisch ein Steuergerät eines Herzunterstützungssystems nach dem Stand der Technik und
- Figur 2:: ein Steuergerät eines Herzunterstützungssystems gemäß der Erfindung.

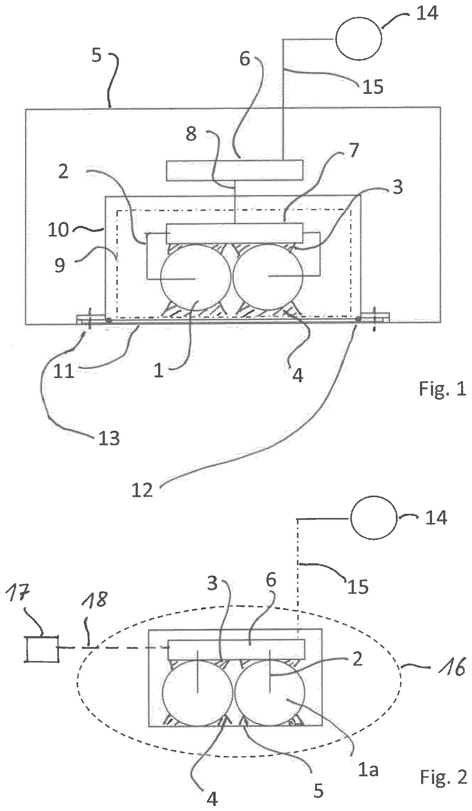

Für ein Herz-Unterstützungssystem, beispielsweise ein ventrikuläres Herzunterstützungssystem (VAD-System) wird nach dem Stand der Technik entsprechend Fig.1 wie folgt geplant: Es wird eine tragbare Steuereinheit mit einem Gehäuse 5 vorgesehen. Diese kann über eine Leitung 15 eine symbolisch dargestellte Blutpumpe 14 steuern und mit der notwendigen Energie versorgen. Die Blutpumpe ist implantierbar, wobei die Steuereinheit sowohl implantierbar als auch extrakorporal vorgesehen sein kann. Zur Erzielung der geplanten Laufzeit sind 4 Lithium-Ionen-Akkuzellen 1 mit hoher Energiedichte, im Format 18650 vorgesehen, wobei jede Zelle über eine Kapazität von 3000 mAh bei typisch 3,6 V Zellspannung verfügen soll. In der als Steuereinheit ausgebildeten Funktionseinheit ist für diese 4 Lithium-lonen-Akkuzellen innerhalb des AkkuPacks und seiner Umhüllung 9 ein Batteriemanagementsystem 7 in der Größe von 65 mm x 72 mm, mit einer Dicke von 3 mm vorgesehen. Die 4 Akkuzellen sind mit dem Batteriemanagementsystem zusammen in dem Akku-Pack mit einem Akku-Pack-Gehäuse oder einer Umhüllung 9 integriert, welches eine Größe von ca. 74 mm x 68 mm und eine Dicke von 23 mm aufweist. Elektrische Anschlüsse der Akkuzellen sind innerhalb des Akkupacks über Anschlussleitungen 2 mit dem Batteriemanagementsystem 7 verbunden. Dieses Akku-pack ist mit einem nur symbolisch dargestellten Kabelschwanz 8 mit Steckverbinder versehen, mit welchem es auf der Hauptleiterplatte der Anwendungselektronik 6 des Steuergeräts kontaktiert wird. Um das Akku-Pack in das Gehäuse 5 der Steuereinheit des VAD-Systems einsetzen zu können, wird im Gehäuse ein extra hierfür angefertigter Akkuschacht 10 vorgesehen, der über einen wasserdicht abschließbaren Deckel 11 mit entsprechenden Dichtungen 12 und Befestigungselementen13 verfügt. Als Befestigungselemente können beispielsweise Schrauben mit einem unüblichen Kopf vorgesehen sein, die mit einem handelsüblichen Schraubendreher nicht lösbar sind. Hierzu kann sich eine im Querschnitt polygonale Vertiefung eignen, für die ein passendes Spezialwerkzeug nur an einen ausgewählten Personenkreis geliefert wird.

Das Akku-Pack mit den beschriebenen Lithium-Ionen-Akkus kann nicht sterilisiert werden. Daher muss der Lithium-Ionen-Akku mit 4 Akkuzellen so konstruiert sein, dass er nach der Herzoperation zur Implantation der Herzpumpe in die bis dahin sterile Steuereinheit eingebaut werden kann. Der Akkuschacht 10 erfordert im Gehäuse 5 der Steuereinheit ein Volumen von ca. 78 mm x 72 mm x 27 mm zuzüglich des Montageplatzes für das Kabel zum Batteriemanagementsystem. Hinzu kommt der Platzbedarf für den Deckel der demontierbar und mit einer wasserdichten Dichtung umgeben ist. Das Akku-Pack kann in dem Batterieschacht durch dort vorgesehene Federn kontaktierbar sein. Alles in allem ergibt sich nach dem Stand der Technik ein benötigtes Volumen im Gehäuse von ca. 170 cm3. Der Zwischenraum zwischen den zylindrischen Ackuzellen ist für die Elektronik der Steuereinheit/Anwendungselektronik nicht nutzbar, da er durch mehrere Zwischenwände von der Elektronik auf der Haupt-Leiterplatte der Anwendungselektronik getrennt ist.

Die Akkuzellen 1 können mit dem Batteriemanagementsystem 7 mittels einer Klebeverbindung 3 verklebt sein. Ebenso können die Akkuzellen 1 auch durch eine Klebeverbindung 4 mit der Umhüllung 9 des Akku-Pack verbunden sein.

Die erfindungsgemäße Umsetzung des funktional gleichen Herzunterstützungssystems soll an genau diesem Beispiel in der Fig.2 erläutert werden, wobei jeweils gleiche Bezugszeichen funktional gleiche Einheiten in beiden Figuren bezeichnen.

Um die gleiche Akku-Laufzeit wie mit den oben beschriebenen Lithium-Ionen Akkus zu erreichen, werden vier Natrium-Ionen-Zellen 1a der Baugröße 26700 mit jeweils 3300 mAh und einer typischen Zellspannung von 3,1V gewählt. Da die Natrium-Ionen-Akkus tiefentladen werden dürfen, können diese nach der Erstinbetriebnahme im Herstellerwerk wieder vollständig entladen werden. Sie können dann in der Folge innerhalb der Steuereinheit sterilisiert werden und jahrelang in der Sterilverpackung der Steuereinheit oder des Herzunterstützungssystems ohne Überwachung verbleiben. In der Figur 2 ist gestrichelt symbolisch die Sterilverpackung 16 dargestellt, die die Form eines hermetische verschlossenen Kunststoffbeutels haben kann. Beim Laden und bei der aktiven Verwendung der Funktionseinheit und der Akkumulatoren ist dann die Sterilverpackung im Regelfall nicht mehr vorhanden. Bei der Erstbenutzung werden die Natrium-Ionen-Akkus aufgeladen und stehen dann einsatzbereit zur Verfügung. Die übliche Lebensdauer der Natrium-Ionen-Akkus beträgt 10 bis 15 Jahre. Die typische Einsatzdauer einer Steuereinheit für ein VAD-System beträgt 3 Jahre. Somit gibt es keine Notwendigkeit, den Natrium-Ionen-Akkumulator innerhalb der Nutzungsdauer der Steuereinheit, jemals zu wechseln. Die aus vier Zellen bestehende Natrium-Ionen-Akku-Einheit benötigt kein Batteriemanagementsystem. Die einzelnen Natrium-Ionen-Akku-Zellen 1a können mit ihren elektrischen Anschlüssen 2 in Form von Lötfahnen direkt auf der Haupt-Leiterplatte bestückt werden, die die Anwendungselektronik 6 bildet oder Teil der Anwendungselektronik/des Steuergerätes ist. Auch der Zwischenraum zwischen den Akku-Rundzellen 1a kann teilweise genutzt werden, sei es für Elektronik auf der Hauptleiterplatte oder für Verstärkungsrippen im Gehäuse. In der Fig. 2 ist dargestellt, dass dieser Zwischenraum zwischen den Akkuzellen einerseits durch Klebverbindungen 3, 4 genutzt wird, um einerseits die Lötstellen zwischen Akku und Anwendungselektronik mechanisch zu entlasten und andererseits durch Klebverbindungen zwischen den Akkus und dem Gehäuse 5 die mechanische Steifigkeit des Gehäuses zu vergrößern. Die vier Natrium-Ionen-Rundzellen vom Format 26700 belegen in der Steuereinheit ein Volumen von zusammen 149 cm3.

Eine Verbindung der Natrium- lonen-Akkus mit einer Ladespannungsquelle 17 kann, wie in der Figur 2 gezeigt ist, über die Platine der Anwendungselektronik und einen elektrischen Anschluss hergestellt werden. Diese Platine kann Anschlüsse aufweisen, die mit der externen Ladespannungsquelle beispielsweise mittels einer Steckverbindung 18 oder mittels eines induktiven Ladekreises verbindbar sind. Die Anwendungselektronik kann einen Laderegler aufweisen, der ohne Zwischenschaltung von Schutzvorrichtungen mit den Natrium-lonen-Akkumulatoren verbunden ist. Schutzvorrichtungen sind zu diesem Zweck auf der Platine nicht vorgesehen. Die Anschlussleitung /Steckverbindung 18 ist in der Figur 2 gestrichelt eingezeichnet, da die Ladeverbindung im Regelfall nur dann genutzt wird, wenn die Sterilverpackung 16 geöffnet oder entfernt ist.

Somit ergibt trotz der geringeren netto-Energiedichte der Natrium-lonen-Akku-Zellen gegenüber den Lithium-lonen-Akku-Zellen in der Summe beim Einsatz von Natrium-lonen-Akku-Zellen eine Volumenreduktion im eine medizinische Funktionseinheit bildenden tragbaren Steuergerät. In ähnlicher Art und Weise reduziert sich auch das Gewicht.

Da das Steuergerät mit den Natrium lonen- Akkus in sterilisierter Form einfach gelagert und transportiert werden kann, ergibt sich auch bei der Logistik eine erhebliche Vereinfachung.

Als besonders vorteilhaft erweist sich auch, dass die Rohstoffe, welche für den Aufbau von Natrium-Ionen Akkus benötigt werden, praktisch grenzenlos zur Verfügung stehen. Natrium ist in Form von Natriumchlorid in den Weltmeeren praktisch unbegrenzt vorhanden. Auch die Umweltbelastung wird somit bei Verwendung von Natrium- lonen- Akkus merklich reduziert.

## Patentansprüche

1. Medizinische Funktionseinheit mit einer elektrischen Energieversorgung, die einen oder mehrere aufladbare Natrium-Ionen-Akkumulatoren (1a) aufweist, **dadurch gekennzeichnet, dass** die Energieversorgung und die Natrium- lonen- Akkumulatoren weniger als sechs der folgenden sechs Schutzvorrichtungen oder weniger als 5 oder weniger als 4, weniger als drei, oder weniger als zwei der folgenden sechs Schutzvorrichtungen (7) aufweisen:
Ladestromschutz,
Laststromschutz,
Unterspannungsschutz,
Überladungsschutz,
Hochtemperaturschutz,
temperaturabhängige Lastrombegrenzung.

2. Medizinische Funktionseinheit nach Anspruch 1, **dadurch gekennzeichnet, dass** die Kathode oder die Kathoden und die Anode oder die Anoden der Natrium- Ionen- Akkumulatoren (1a) unmittelbar mit einer Ladespannungsquelle (17) verbunden oder verbindbar sind.

3. Medizinische Funktionseinheit nach Anspruch 2, **dadurch gekennzeichnet, dass** die Ladespannungsquelle (17) durch einen induktiven Ladekreis gebildet ist.

4. Medizinische Funktionseinheit nach Anspruch 1, 2 oder 3, **dadurch gekennzeichnet, dass** die Kathode oder die Kathoden und die Anode oder die Anoden der Natrium- lonen- Akkumulatoren (1a) unmittelbar mit einem elektrischen Aggregat (14) verbunden oder verbindbar sind, das mit der Energie aus dem oder den Natrium- lonen- Akkumulatoren betreibbar ist.

5. Medizinische Funktionseinheit nach einem der Ansprüche 1 bis 4, **dadurch gekennzeichnet, dass** die Energieversorgung und die Natrium-lonen- Akkumulatoren (1a) keine der folgenden sechs Schutzvorrichtungen (7) aufweisen:
Ladestromschutz,
Laststromschutz,
Unterspannungsschutz,
Überladungsschutz,
Hochtemperaturschutz,
temperaturabhängige Lastrombegrenzung,
und dass die Kathode oder die Kathoden und die Anode oder die Anoden der Natrium- lonen- Akkumulatoren unmittelbar mit einer Ladespannungsquelle (17) verbunden oder verbindbar sind.

6. Medizinische Funktionseinheit nach einem der Ansprüche 1 bis 5, **dadurch gekennzeichnet, dass** die Energieversorgung und die Natrium-lonen- Akkumulatoren (1a) keine der folgenden sechs Schutzvorrichtungen (7) aufweisen:
Ladestromschutz,
Laststromschutz,
Unterspannungsschutz,
Überladungsschutz,
Hochtemperaturschutz,
temperaturabhängige Lastrombegrenzung
und dass die Kathode oder die Kathoden und die Anode oder die Anoden der Natrium- lonen- Akkumulatoren unmittelbar mit einem elektrischen Aggregat (14) verbunden oder verbindbar sind, das mit der Energie aus dem oder den Natrium- lonen- Akkumulatoren betreibbar ist.

7. Medizinische Funktionseinheit nach einem der Ansprüche 1 bis 6, **dadurch gekennzeichnet, dass** die Energieversorgung und die Natrium-lonen- Akkumulatoren (1a) keine Schutzvorrichtung aufweisen, die während einer Lagerung Messgrößen der Natrium- lonen- Akkumulatoren erfasst und/oder ein Warnsignal bei Unterschreiten einer Spannungsschwelle abgibt oder abspeichert und/oder dass die medizinische Funktionseinheit Teil eines ventrikulären Herzunterstützungsgerätes ist.

8. Medizinische Funktionseinheit nach Anspruch 7, **dadurch gekennzeichnet, dass** das ventrikuläre Herzunterstützungsgerät einen implantierbaren Teil (14) und einen extrakorporalen Teil (5) aufweist und dass die Natrium- lonen- Akkumulatoren (1a) in dem extrakorporalen Teil angeordnet sind oder dass die Natrium- lonen- Akkumulatoren in dem implantierbaren Teil angeordnet sind.

9. Medizinische Funktionseinheit nach einem der Ansprüche 1 bis 8, **dadurch gekennzeichnet, dass** die Natrium- lonen- Akkumulatoren (1a) mittels eines oder mehrerer Steckverbinder (8) mit dem elektrischen Teil (6) der medizinischen Funktionseinheit verbindbar sind oder dass die Natrium- lonen- Akkumulatoren (1a) in einen Batterieschacht (10) der medizinischen Funktionseinheit einsetzbar und in diesem mittels federnder Druckkontakte kontaktierbar sind.

10. Medizinische Funktionseinheit nach einem der Ansprüche 1 bis 9, **dadurch gekennzeichnet, dass** die Natrium- lonen- Akkumulatoren (1a) in einem Gehäuse (5) der medizinischen Funktionseinheit fest verbaut sind.

11. Medizinische Funktionseinheit nach einem der Ansprüche 1 bis 10, **gekennzeichnet durch** eine die Funktionseinheit (5, 6) gemeinsam mit einem oder mehreren Natrium- lonen-Akkus (1a) einschließende Sterilverpackung (16).

12. Ventrikuläres Herzunterstützungssystem mit einer medizinischen Funktionseinheit (5, 6) nach einem der Ansprüche 1 bis 11.

13. Verfahren zur Sterilisation eines oder mehrerer Natrium- lonen-Akkumulatoren **dadurch gekennzeichnet, dass** der oder die Natrium-lonen- Akkumulatoren (1a) zunächst vollständig entladen und nach dem Entladen sterilisiert werden.

14. Verfahren nach Anspruch 13, **dadurch gekennzeichnet, dass** zur Sterilisation einer Funktionseinheit (5, 6) mit einem oder mehreren Natrium-lonen-Akkumulatoren (1a) die Natrium- lonen- Akkumulatoren, mit denen die Funktionseinheit betrieben wird, vor oder nach dem Einsetzen in die Funktionseinheit vollständig entladen werden und dass nach dem Entladen der Natrium- lonen- Akkumulatoren die Funktionseinheit gemeinsam mit den Natrium- lonen- Akkumulatoren sterilisiert wird.

15. Verfahren nach Anspruch 13 oder 14 **dadurch gekennzeichnet, dass** zur Sterilisation einer, insbesondere implantierbaren, medizinischen Funktionseinheit (5, 6, 14) die Natrium- lonen- Akkumulatoren (1a), mit denen die medizinische Funktionseinheit betrieben wird, vor oder nach dem Einsetzen oder dem festen Einbau in die medizinische Funktionseinheit vollständig entladen werden und dass nach dem Entladen der Natrium- lonen- Akkumulatoren die medizinische Funktionseinheit gemeinsam mit den Natrium- lonen- Akkumulatoren sterilisiert wird.
